# EUROPEAN PATENT SPECIFICATION

(11) **EP 1 759 650 B1**
(45) Date of publication and mention of the grant of the patent: **24.03.2010**
(21) Application number: 06254417.6
(22) Date of filing: 23.08.2006
(51) Int. Cl.: A61B 17/78

(54) **Intramedullary nail assembly**
Marknagel-System
Ensemble clou intramedullaire

(30) Priority: 31.08.2005 US 217180
(43) Date of publication of application: 07.03.2007
(73) Proprietor: DePuy Products, Inc., Warsaw, IN 46581 (US)
(72) Inventor: Wallace, Matthew S., Warsaw, IN 46845 (US); Haidukewych, George J., Tampa, FL 33647 (US); Ducret, Frederic, Le Locle (CH)
(74) Representative: Gover, Richard Paul

(56) References cited:
- EP-A2- 0 441 577
- EP-A2- 1 175 872
- WO-A-2004/100810
- DE-U1- 29 502 722
- GB-A- 2 209 947
- US-B1- 6 221 074
- US-B1- 6 235 031

## Description

The present invention relates generally to the field of orthopaedics, and more particularly, to a device for securing a prosthetic component to bone for use in with orthopaedic trauma or orthopaedic joint products.

The skeletal system includes many long bones that extend from the human torso. These long bones include the femur, fibula, tibia, humerus, radius and ulna. These long bones are particularly exposed to trauma from accidents, and as such often are fractured during such trauma ad may be subject to complex devastating fractures.

Automobile accidents, for instance, are a common cause of trauma to long bones. In particular, the femur and tibia frequently fracture when the area around the knee is subjected to a frontal automobile accident.

Often the distal end or proximal portions of the long bone, for example the femur and the tibia, are fractured into several components and must be realigned. Mechanical devices, commonly in the forms of pins, plates, screws, nails, wires and external devices are commonly used to attach fractured long bones. The pins, plates, wires, nails and screws are typically made of a durable material compatible to the human body, for example titanium, stainless steel or cobalt chromium.

Fractures of the long bone are typically secured into position by at least one of three possible techniques.

The first technique for fixing segments of a fractured bone involves the use of intramedullary nails that are positioned in the intramedullary canal of those portions of the fractured bone.

A second technique for fixing segments of a fractured bone involves the use of internal bone plates that are positioned under the soft tissue and on the exterior of the bone and bridges the fractured portion of the bone.

A third technique for fixing segments of a fractured bone involves the use of external fixators. These external fixators have at least two general categories. In one category the fixator is generally linear with a first portion of the fixator to connect to a first fracture segment of the bone and a second fracture segment of the fixator to connect to the second fracture segment of the bone. A first series of bone screws or pins are first connected to the fixator and then into the first portion of the bone. Then a second series of screws or pins are connected to the fixator and then to the second fracture segment of the bone, thereby securing the first portion fracture segment of the bone to the second portion of the bone.

A fourth technique for fixing segments of a fractured bone uses a ring type fixator with a series of spaced-apart rings to secure the bone. For example, an upper ring and a lower ring are spaced apart by rods. A plurality of wires is placed through the long bone and is connected on each end of the long bone by the ring. The wires are then tensioned much as a spoke in a bicycle are tightened, thereby providing for a rigid structure to support the first fracture segment portion of the bone. Similarly, a plurality of wires are positioned through the second fracture segment of the bone and are secured to and tensioned by the lower ring to provide a rigid fixation of the second fracture segment of the bone bridging the fracture site.

There are a variety of devices used to treat femoral fractures. Fractures of the neck, head or intertrochanter of the femur have been successfully treated with a variety of compression screw assemblies which include generally a compression plate having a barrel member, a lag screw and a compressing screw. The compression plate is secured to the exterior of the femur and the barrel member is inserted into a pre-drilled hole in the direction of the femoral head.

The lag screw which has a threaded end and a smooth portion is inserted through the barrel member so that it extends across the break and into the femoral head. The threaded portion engages the femoral head. The compressing screw connects the lag screw to the plate. By adjusting the tension of the compressing screw the compression (reduction) of the fracture can be adjusted. The smooth portion of the lag screw must be free to slide through the barrel member to permit the adjustment of the compression screw.

Subtrochanteric and femoral shaft fractures have been treated with the help of intramedullary rods which are inserted into the marrow canal of the femur to immobilize the femur parts involved in fractures. A single angled cross-nail or locking screw is inserted through the femur and the proximal end of the intramedullary rod. In some varieties, one or two screws may also be inserted through the femoral shaft and through the distal end of the intramedullary rod. The standard intramedullary rods have been successfully employed in treating fractures in lower portions of the femoral shaft.

Trochanteric nails for use in preparing femoral neck fractures utilize a screw in the form of, for example, a lag screw. The lag screws have several different problems in use that are generally related to the lag screw not remaining in the proper position with respect to intramedullary nail during the operating live of an implant. For example, the lag screw may cut proximally through the femoral neck and head causing the neck and head to move out its operating position in cooperation with the acetabulum. Such a movement is render the patient non-ambulatory. Another issue that may occur with lag screws is medial migration of a lag screw through the femoral head and into the pelvic cavity. Yet another issue with an intramedullary nail lag screw is lateral migration or lateral pullout of the screw from the long bone.

Yet another problem with lag screws in trochanteric nail applications is the problem of neck collapse. Early after the implantation of the trochanteric nail, for example, at the first weight-bearing instance of the patient, the head of the femur may move distally due to a phenomenon known as neck collapse. If the lag screw does not capture enough cancellous bone in the femoral neck, the neck and head may move laterally causing the phenomenon known as neck collapse and creating a leg length and other issues for the patient.

Medial migration is only one of many problems that occur with a fastener for use with orthopaedic prosthetic components. The design of fasteners in cancellous and/or osteoporotic bone must deal with parameters such as pull-out forces, installation torque requirements, stripping of the bone, migration and others.

The proximal femoral fractures, for example, those around the less trochanter, greater trochanter, and femoral neck have been successful treated with a variety of compression screw assemblies and intramedullary rods. The intramedullary rods are inserted into the narrow canal of the femur to immobilize the femur parts involved in the fracture. Typically, a single screw is inserted through the femur and the proximal end of the intramedullary rod. Alternatively, a second screw may be inserted through the femur and into the proximal end of the intramedullary rod to prevent rotation of, for example, the neck and head of the femur.

One of the earliest intramedullary devices introduced in the United States was the nail sold by Howmedica Company under the trade mark Grosse-Kempf. The Grosse-Kempf nail includes a threaded hole in the intramedullary rod for receiving an interlocking screw. The fully threaded screw can not slide in order to permit the compression found in typical compression screw assemblies.

Another known device is disclosed in US-3433220, sold under the trade mark Zickel. The Zickel nail is a solid intramedullary nail having a single proximal tri-flange cross-nail which is inserted into the direction of the femoral head. The solid cross-section does not permit the nail to be introduced over a guide rod. Thus, the nail is prevented from being used for comminuted and distal fractures of the femur because the closed surgical technique cannot be practised. In addition, adequate compression cannot be achieved due to the requirement to lock cross-nail.

Yet another known interlocking nail is sold by Smith & Nephew Richards Inc under the trade mark Russell-Taylor. The Russell-Taylor nail similarly requires a fully threaded locking screw and therefore does not permit sliding of the screw relative to the intramedullary rod.

Yet a further known nail device is that sold under the trade mark Gamma by Stryker-Howmedica. The Gamma nail provides for sliding compression of the lag screw through the use of a smooth shaft. The Gamma nail stops rotation of the lag screw by means of a set screw through the proximal portion of the intramedullary nail.

A further known nail device is that sold by DePuy Orthopaedics Inc under the trade mark Ace Trochanteric, which provides for means of stopping rotation of the femoral head in an unstable fracture pattern by the use of a second threaded screw in the femoral head. The lag screw is permitted to rotate freely within the nail.

EP-1175872 discloses an intramedullary nail and lag screw. The lag screw is arranged to connect a bone portion separated from the femur by fracture to the main portion of the femur. The lag screw is fixedly secured in the separated bone portion and extends through the main portion of the femur. An intramedullary nail member is installed in the femur in order to fix the lag screw to the femur. The lag screw is secured in the nail member by a set screw inserted into an outer end of the nail member. In order to prevent the rotation of the separated bone portion about the lag screw, an auxiliary connector is inserted through the nail member between the lag screw and the set screw. The lag screw is clamped by the set screw via a spacer which transmit a clamping force onto the lag screw.

US-6221074 discloses a femoral intramedullary rod system capable of treating a variety of femoral bone fractures using a uniform intramedullary rod design. The system generally comprises an intramedullary rod which defines an opening having an outer surface and a transverse member including a bone engaging portion and a connection portion defining a through hole with the nail sized to pass therethrough. A pin is selectively coupled to the transverse member to rigidly assemble the transverse member to the nail when the nail is passed through the through hole and the pin is received within the opening.

The present invention is directed to alleviate at some of the aforementioned concerns with orthopaedic fasteners. The scope of the present invention is defined by the appended claims.

The present invention provides apparatus for treating fractures of the proximal femur and includes a screw, a proximal sleeve, and an intramedullary rod. The screw has a threaded portion, a smooth portion, a flat portion, and a ridged portion. The proximal sleeve fits around the proximal portion of the nail with an extended tab that can lock the screw. The rod has a head and a stem. There is a least one opening through the head of the rod in an angled direction toward the femoral head relative to the longitudinal axis of the rod. The opening is adapted to receive the screw to permit the threaded portion of the screw to engage the femoral head. The flat and ridged portion of the screw interface with the extended tab of the proximal sleeve to allow for locking of the screw.

The present invention provides apparatus for treating fractures of the proximal femur which marry the fixation attributes of an intramedullary nail with the proven benefits of the sliding compression screw. Additionally, the present invention allows for the surgeon to choose between locking the screw from sliding and rotating, locking the screw from rotation only, or not locking the screw at all.

The proximal sleeve may fit on the proximal end of the intramedullary rod. The extended tab of the proximal sleeve may fit unto either of the ridges or the flat section of the lag screw in order to allow the surgeon to lock the screw. The screw fits through the opening of the intramedullary rod.

According to one embodiment of the present invention, there is provided an intramedullary nail assembly for use in a medullary canal of a long bone. The assembly includes a nail for positioning at least partially in the medullary canal. The nail defines an aperture through the nail. The nail assembly also includes a screw. The screw can be fitted into the aperture of the nail. The screw has a shank defining an end and a periphery of the shank. A portion of the periphery defines a thread. The nail assembly also includes means for fixedly securing the screw to the nail.

According to another embodiment of the present invention there is provided an intramedullary nail assembly for use in a medullary canal of a long bone. The assembly includes a nail for positioning at least partially in the medullary canal. The nail defines an aperture through the nail. The assembly also includes a screw which can be positioned in the aperture of the nail. The screw has a shank defining an end and a periphery of the shank. A portion of the periphery defines a thread. The assembly also includes a locking component operably connected to the screw and to the nail for fixedly securing the screw to the nail.

According to yet another embodiment of the present invention there is provided an intramedullary nail assembly for use in a medullary canal of a long bone. The assembly includes a nail for positioning at least partially in the medullary canal. The nail defines an aperture through the nail. The assembly also includes a fastener which is positioned in the aperture of the nail. The fastener is adapted for attachment to the long bone. The assembly also includes a sleeve fitted at least partially over the nail and operably connected to the screw.

According to another embodiment of the present invention there is provided a screw for use with an intramedullary nail for use in a medullary canal of a long bone. The screw includes a shank defining an end and a periphery of the shank. A portion of the periphery defines a thread. The screw also includes a locking feature on the periphery of the shank adapted to lock the screw to the nail.

According to yet another embodiment of the present invention there is provided a locking component for use to secure a screw to an intramedullary nail to form an intramedullary nail assembly for use in a medullary canal of a long bone. The locking component includes a first portion for cooperation with the nail and a second portion. The second portion fixedly secures the locking component to the screw.

According to another embodiment of the present invention there is provided an intramedullary nail for use with a screw and a sleeve to repair a fractured long bone. The nail is used in a medullary canal of a long bone. The nail includes a body defining an external periphery of the body and a transverse aperture through the body for receiving the nail. The body further defines a portion of the external periphery of the body for receiving the sleeve.

Nails provided by the present invention can be used in a method for performing trauma surgery on a long bone, which includes the step of providing an intramedullary nail. The nail defines an aperture through the nail. The method also includes the steps of positioning the nail at least partially in the medullary canal and providing a screw for attachment to the long bone.

A preferred method for performing trauma surgery on a long bone comprises the steps of:
providing an intramedullary nail, said nail defining an aperture therethrough;
positioning the nail at least partially in the medullary canal;
providing a screw for attachment to the long bone, said screw having a first position for fixedly attaching the screw to the nail and having a second position for slidingly attaching the screw to the nail;
selecting one the first position and the second position of the screw corresponding to one of fixed attachment and sliding attachment of the screw to the nail and
positioning the screw in the aperture of the nail in the one of the first position and the second position.

The screw has a first position for fixedly attaching the screw to the nail and a second position for slidingly attaching the screw to the nail. The method further includes the steps of selecting the first position or the second position of the screw corresponding to fixed attachment or sliding attachment of the screw to the nail and positioning the screw in the aperture of the nail in the one of the first position and the second position.

The technical advantages of the present invention include the ability to provide an intramedullary nail assembly where by the surgeon may choose between a screw that slides along the nail aperture and the screw that is locked to the intramedullary nail.

For example, according to one aspect of the present invention, the intramedullary nail assembly is provided for use in an intramedullary canal of a long bone. The assembly includes a nail for positioning at least partially in the medullary canal and a screw positioned in the aperture of the nail. The screw has a shank portion defining a periphery and an end. A portion of the periphery defines a thread. The nail assembly further includes a means for securing the screw to the nail. The means, for example, may be in the form of, a transverse groove formed on the periphery of the shank of the screw. The transverse groove may cooperate with, for example, a locking component in the form of a sleeve. The sleeve and the groove provide an ability to lock the screw with respect to the nail. If the sleeve is not used, the screw can slide and if the sleeve cooperates with the groove the nail may be locked to prevent sliding.

Thus, the present invention provides for the ability to provide an intramedullary nail assembly in which the surgeon may choose between the sliding and locking of the screw with respect to the nail.

The technical advantages of the present invention further include the ability to provide an intramedullary nail assembly in which the surgeon may choose a locked configuration between the screw and the nail and a sliding and rotating relationship between the screw and the nail. For example, according to another aspect of the present invention an intramedullary nail assembly is provided for use in a medullary canal of a long bone. The nail assembly includes a nail for positioning partially in the canal and a screw positioned in the aperture of the nail.

The nail assembly further includes a means for securing the screw to the nail. The means for securing the screw to the nail may be in the form of, for example, a sleeve slidably fitted over the nail and a transverse groove formed on the periphery of the screw. The sleeve includes a locking component in the form of, for example, a tab which cooperates with the transverse groove on the screw. The sleeve and the screw serve to be locked into position with respect to the nail. Alternatively, the sleeve can be omitted, permitting the screw to slide and rotate. Thus, the present invention provides the surgeon the ability to choose between having a screw cooperate with a nail in a locked relationship or in a sliding and rotating relationship.

The technical advantages of the present invention also include the ability to provide an intramedullary nail assembly in which the surgeon can selectively change from the sliding and rotating of the screw within the nail to the sliding only of the screw within the nail.

For example, according to another aspect of the present invention, an intramedullary nail assembly is provided for use in the medullary canal of a long bone. The nail assembly includes a nail and a screw positioned in the aperture of the nail. The nail assembly further includes means for securing the screw to the nail in the form of, for example, a sleeve.

The screw includes a feature in the form of, for example, a tab on the sleeve to permit sliding motion of the screw of the nail. If the sleeve is removed, then the screw may have sliding and rotating motion with respect to the nail. Thus, the present invention provides for an intramedullary nail assembly in which the surgeon may choose between a sliding and rotating mode for the screw within the nail and a sliding only mode for the screw within the nail.

The technical advantages of the present invention further include the ability to treat a variety of fractures. For example, according to another aspect of the present invention, an intramedullary nail assembly is provided including a nail and a screw positioned in the aperture of the nail as well as mean for securing the screw to the nail. The screw may include a flat as well as a transverse groove for cooperation with, for example, a sleeve fitted over the nail. The surgeon my not utilizing the sleeve, using the sleeve in cooperation with the groove or using the sleeve in cooperation with the flat. The sliding, rotating, and locked alternative positions of the screw within the nail permit the nail assembly to be used in a variety of fractures. Thus, the present invention provides for the technical advantages of treating a variety of fractures.

The technical advantages of the present invention further include the ability to provide an intramedullary nail device which combines the superior mechanical and biological attributes of intramedullary fixation with the proven benefits of a sliding compression screw for a fracture reduction. For example, according to yet another aspect of the present invention an intramedullary nail assembly is provided including a nail for positioning at least partially in the medullary canal and a screw positioned in the aperture of the nail. The screw, at least in one mode, is permitted to slide along the aperture to provide for sliding compression. Thus, the present invention provides for an intramedullary nail fixation combination with a sliding compression screw.

The technical advantages of the present invention also include the ability to permit the lag screw to be smooth through the shaft for greater strength. For example, according to yet another aspect of the present invention an intramedullary nail assembly is provided including a nail for positioning in the canal and a screw positioned in the aperture of the nail. The screw includes a shank that is smooth through the shaft. The screw may include ridges and a flat on the screw but such ridges and flats on the screw are on the most lateral end of the screw. The largest forces on the screw are proximal, not lateral thus the smooth lateral portion of the lag screw increases the strength of the screw. Thus, the present invention provides for a lag screw that is smooth through shaft for greater strength.

The technical advantages of the present invention further include the ability to provide for an intramedullary nail in which the surgeon may permit locking of the screw onto the nail. For example, according to yet another aspect of the present invention an intramedullary nail assembly is provided including a nail that may be positioned in the canal and a screw positioned in the aperture of the nail. The nail assembly further includes means for securing the screw to the nail. For example, the means may be in the form of a sleeve including a tab. The tab of the sleeve cooperates with, for example, a transverse groove in the most lateral end of the screw. The tab being secured in the grooves of the screw permit the complete locking of the screw. Thus, the present invention provides for the use of an intramedullary nail which has complete locking of the screw.

The technical advantages of the present invention further include the ability to provide an intramedullary nail assembly in which the surgeon may select sliding of the compression screw without rotation. For example, according to yet another aspect of the present invention an intramedullary nail assembly is provided including an intramedullary nail for positioning in the medullary canal and a screw positioned in the aperture of the nail. The nail assembly further includes means for securing the screw to the nail in the form of, for example, a sleeve fitted over the nail. The sleeve may include a tab that cooperates with a flat formed on a lateral portion of the screw. The tab and the flat on the screw permit the screw to slide within the aperture of the nail without rotation. Thus, the present invention provides for an intramedullary nail assembly that permits sliding without rotation of the screw.

Embodiments of the invention will now be described by way of example with reference to the accompanying drawings, in which:
FIG. 1 is an plan view of a intramedullary nail assembly in accordance with an embodiment of the present invention;
FIG. 2 is an exploded plan view of the intramedullary nail assembly of FIG. 1;
FIG. 3 is a plan view of the lag screw of the intramedullary nail assembly of FIG. 1;
FIG. 4 is a is a cross-sectional view of the lag screw of FIG. 3 showing the head in greater detail;
FIG. 5 is a is a partial cross-sectional view of the lag screw of FIG. 3;
FIG. 6 is an enlarged plan view of the lag screw of FIG. 3;
FIG. 7 is a partial enlarged cross-sectional view of a box shaped thread of the lag screw of FIG. 3;
FIG. 7B is a partial view of an alternate thread form for a lag screw for use with an intramedullary nail assembly of the present invention;
FIG. 7C is a partial view of yet another alternate thread form for a lag screw for use with an intramedullary nail assembly of the present invention;
FIG. 7D is a partial view of an a further thread form for a lag screw for use with an intramedullary nail assembly of the present invention;
FIG. 7E is a partial view of an another thread form for a lag screw for use with an intramedullary nail assembly of the present invention;
FIG. 7F is a partial view of yet another thread form for a lag screw for use with an intramedullary nail assembly of the present invention;
FIG. 7G is a partial view of a further thread form for a lag screw for use with an intramedullary nail assembly of the present invention;
FIG. 8 is a greatly enlarged cross-sectional view of the box shaped thread of FIG. 7;
FIG. 9 is a partial enlarged cross-sectional view of the screw of FIG. 3 showing the grooves in greater detail;
FIG. 10 is a partial enlarged plan view of the lag screw of FIG. 3 showing the flats and grooves in even greater detail;
FIG. 11 is a plan view of the sleeve of the intramedullary nail assembly of FIG. 1;
FIG. 12 is a bottom view of the sleeve of FIG. 11;
FIG. 13 is a side view of the sleeve of FIG. 11;
FIG. 14 is a plan view of the intramedullary nail of the intramedullary nail assembly of FIG. 1;
FIG. 15 is a plan view partially in cross-section of the intramedullary nail of the intramedullary nail assembly with the sleeve and end cap installed;
FIG. 16 is a partial plan view, partially in cross-section, of the intramedullary nail of the intramedullary nail assembly of FIG. 15 with the sleeve and end cap installed;
FIG. 17 is a plan view of the end cap of the intramedullary nail assembly of FIG. 1;
FIG. 18 is a partial plan view, partially in cross-section, of the intramedullary nail assembly of FIG. 1 showing the nail assembly in its first mode of operation permitting sliding and rotation of the lag screw in the transverse opening with the sleeve and end cap removed;
FIG. 19 is a partial plan view, partially in cross-section, of the intramedullary nail assembly of FIG. 1 showing the nail assembly in its second mode of operation permitting only sliding of the lag screw in the transverse opening with the sleeve in cooperation with the flat of the nail;
FIG. 20 is a partial plan view, partially in cross-section, of the intramedullary nail assembly of FIG. 1 showing the nail assembly in its third mode of operation providing fixed securement of the lag screw in the transverse opening with the sleeve in cooperation with the grooves of the nail;
FIG. 21 is an plan view of another embodiment of an intramedullary nail assembly in accordance with the present invention having a nail with an outer periphery corresponding to the outer periphery of the sleeve;
FIG. 22 is a plan view of the intramedullary nail of the intramedullary nail assembly of FIG. 21;
FIG. 23 is a top view of the intramedullary nail of FIG. 22;
FIG. 24 is a partial plan view of yet another embodiment of an intramedullary nail assembly in accordance with the present invention having a cannulated nail with a longitudinal opening extending through the nail;
FIG. 25 is a partial plan view of a further embodiment of an intramedullary nail assembly in accordance with the present invention having a solid nail core;
FIG. 26 is a partial plan view of a yet another embodiment of an intramedullary nail assembly in accordance with the present invention with the nail assembly including additional transverse screws;
FIG. 27 is a partial plan view of a yet another embodiment of an intramedullary nail assembly in accordance with the present invention for use in a humerus; and
FIG. 28 is a flow diagram of a method of performing trauma surgery in accordance with yet another embodiment of the present.

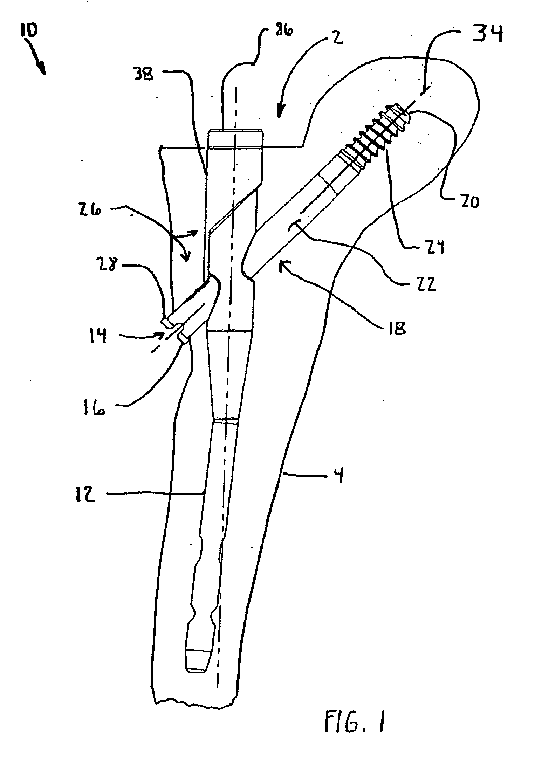

Referring to the drawings, FIG. 1 shows an intramedullary nail assembly 10 for use in the medullary canal 2 of a long bone 4. The long bone 4 may be any long bone of the body, for example, a femur, tibia, or a humerus.

The intramedullary nail assembly 10 includes a nail 12 for positioning at least partially in the medullary canal 2 of the long bone 4. The nail 12 defines an oblique aperture or opening 14. Aperture 14 extends through the nail 12 in a generally oblique direction. A fastener 16 in the form of, for example, a screw may be positioned in the aperture 14 of the nail 12.

The screw 16 includes a shank 18 defining an end 20 and a periphery 22 of the screw. A portion of the periphery 22 defines threads 24. Nail assembly 10 further includes means 26 for fittably securing the screw 16 to the nail 12.

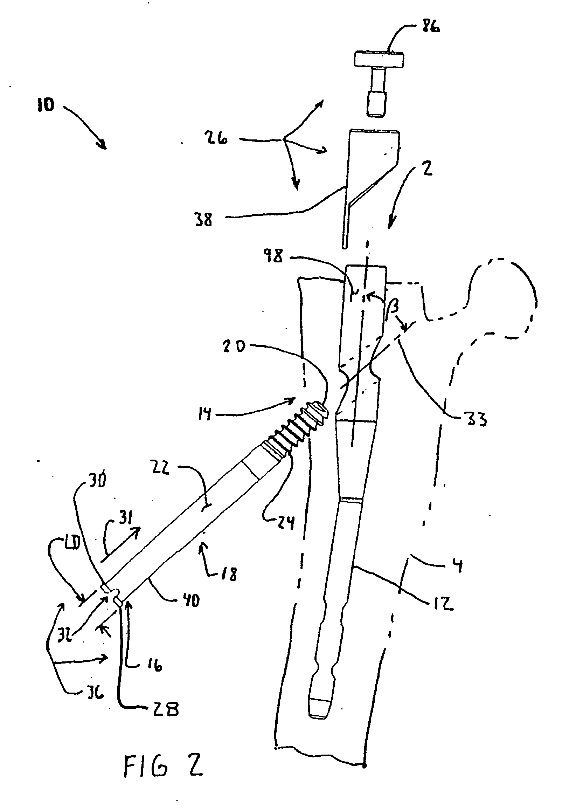

Referring now to FIG. 2, the nail assembly 10 is shown in an exploded view. The screw 16 may be any screw capable of being positioned in the aperture of the nail and of being adapted to be securely fitted to the nail 12. For example and as is shown in FIG. 2, the screw 16 may include a lip 28 extending from the shank 18.

The lip 28 may, as is shown in FIG. 2, extend from second end 30 opposed to first end 20 of the screw 16. The lip 28 may be designed to prevent the screw 16 from migrating in the direction of arrow 31 through the oblique opening 14 of the nail 12. The lip 28 may have any suitable size and shape capable of preventing the screw 16 from transversing out of to the transverse opening 14. For example, the lip 28 may have a lip diameter LD which is larger than the opening diameter OD of the oblique opening 14.

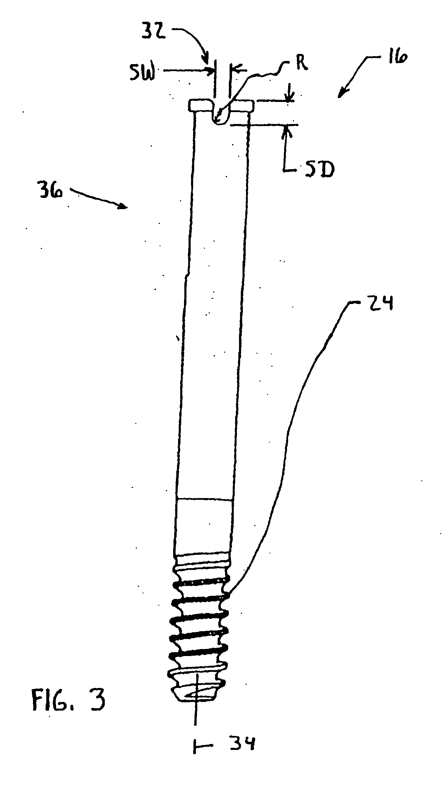

Referring now to FIG. 3, the screw 16 may include or define a rotating feature in the form of, for example, slot 32 formed in the screw 16. The slot 32 may have any suitable size and shape and may, as is shown in FIG. 2, extend from second end 30 of the screw 16. The slot 32 may be utilized to assist in rotating the screw 16 and as such may be centrally located about longitudinal centerline 34 of the screw 16. The slot 32 may have a slot width SW as well as a slot depth of SD. The slot 32 may include a radius R located in the slot 32 to reduce stress risers caused by the slot 32. The slot width SW and slot depth SD are designed to be sufficient for the screw 16 to cooperate with, for example, a screwdriver (not shown) for implanting the screw 16 into the long bone 4.

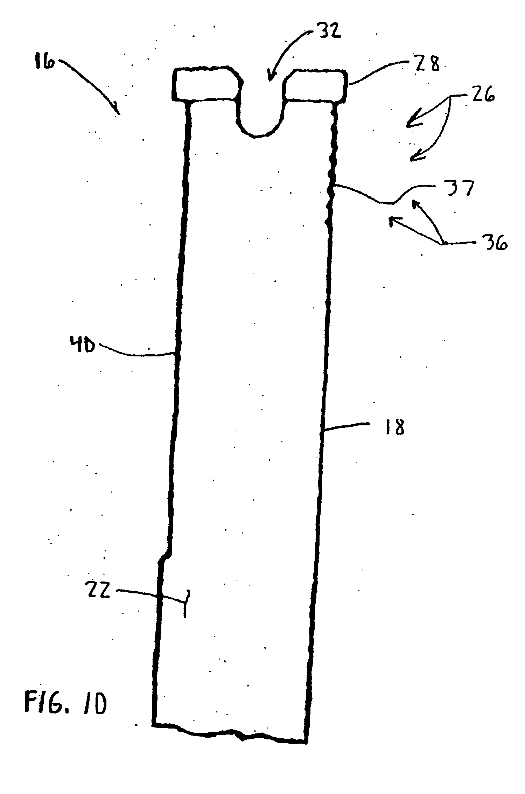

Referring now to FIG. 10, means 26 for securing the screw 16 to the nail 12 may, include a feature 36 located on the periphery 22 on the screw 16. For example, the feature 36 may include, for example, a tooth or teeth 37. The teeth 37 on the screw 16 cooperate with, for example, another component, for example, a sleeve 38 (see FIG. 2) to prevent rotation of the screw 16 with respect to the nail 12.

As shown in FIG. 10, the screw 16 may further define a flat 40 positioned on the periphery 22 of the shank 18 of the screw 16. The flat 40 may as shown in FIG.10 be positioned diametrically opposed to the teeth 37.

Referring now to FIG. 2, the flat 40 cooperates with, for example, the sleeve 38 to permit the sliding motion of the screw 16 along the oblique opening 14 while prohibiting rotation of the screw 16 about the longitudinal centerline 34 of the screw 16.

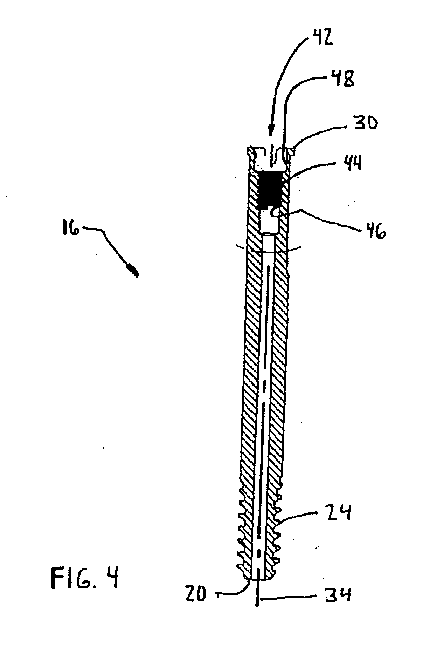

As shown in FIG. 1 and 4, the screw 16 may be cannulated include a longitudinal opening 42 extending along longitudinal centerline 34 of the screw 16. The longitudinal opening 42 may be utilized, for example, for receiving a guide wire (not shown) to guide the screw 16 into position within the transverse opening 14 of the nail 12 and to properly position the screw 16 into the long bone 4.

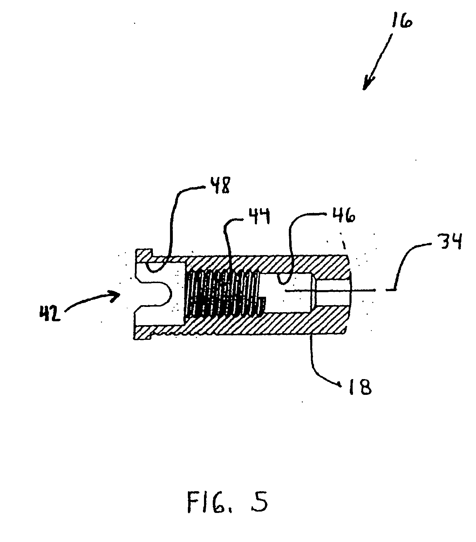

Referring now to FIG. 5, the screw 16 may further include a removal feature 44 in the form of, for example, internal threads formed in the small counter bore 46 formed in the longitudinal opening 42 adjacent the second end 30 of the screw 16. The screw 16 may further include a large counter bore 48 extending from the second end 30 of the lag screw 16 and concentric with the small counter bore 46 as well as with the longitudinal opening 42.

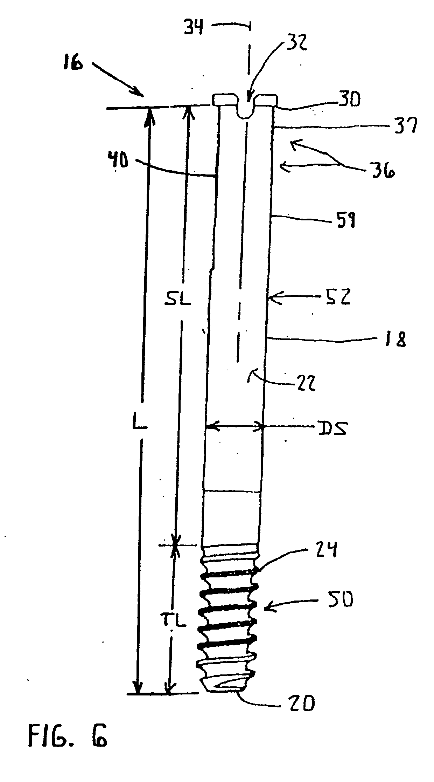

Referring now to FIG. 6, the screw 16 may further include a plurality of threads 24 formed on the shank periphery 22 of shank 18 of the screw 16. The threads 24 may as shown in FIG. 6 have a non-uniform cross-section as disclosed in EP-A-1656899.

Referring again to FIG. 6, the periphery 22 of the shank 18 of the screw 16 includes a first portion 50 into which the threads 24 are formed. It should be appreciated that the first portion 50 may extend along the longitudinal axis 34 of the screw 16 from the first end 20 to second end 30 of the screw 16. It should also be appreciated and as is shown in FIG. 6, that the periphery 22 may include a second portion 52. The second portion 52 of periphery 22 of the shank 18 may define a smooth surface 62. As is shown in FIG. 6, the periphery 22 of the shank 18 may be generally cylindrical and defined by a diameter, for example, DS.

The screw 16 as is shown in FIG. 6, is generally cylindrical and defined by diameter DS and an overall length L. The shank 18 of the screw includes the first portion 50 which include threads 24 and the second portion 52 having the smooth surface 62. The overall length L, is divided into a thread TL and a smooth or unthreaded length SL. The thread length TL defines the first portion 50 and the smooth length SL defines the second portion 52. The thread length TL may, for example, be a portion of, for example, 20 to 40% of the overall length L of the shank 18. It should be appreciated that the smooth length SL is preferably a sufficient length such that the second portion 52 of the screw 16 may be positioned in the oblique opening 14 of the intramedullary nail 12 (see FIG. 1) to permit compression of the bone fracture of long bone or femur 4.

The threads 24 as is shown in FIG. 6, may advance spirally around the periphery 22 of the shank 18 of the screw 16. The threads 24 may be defined by a pitch P defining a spacing along longitudinal axis 34 between adjacent threads. The threads 24 may advance spirally around the longitudinal axis 34 in either a right or a left hand spiral configuration. The threads may, as is shown in FIG. 6, be of a single lead type but may alternatively be double or a triple lead configuration.

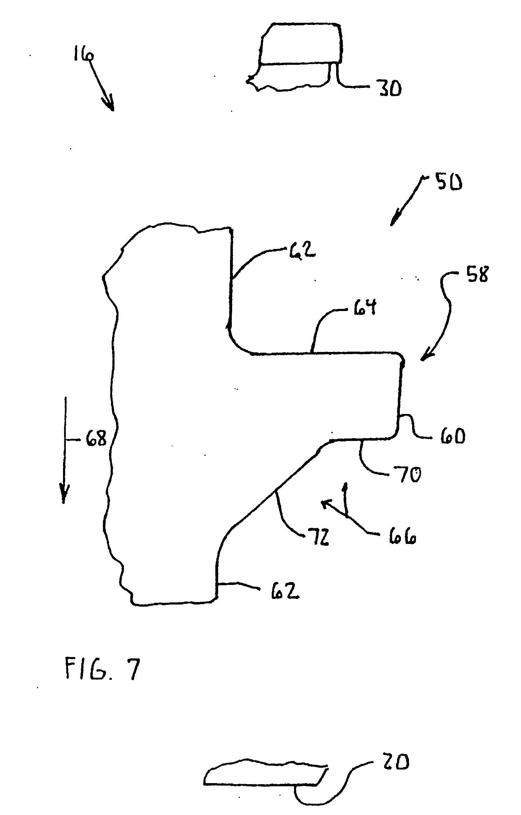

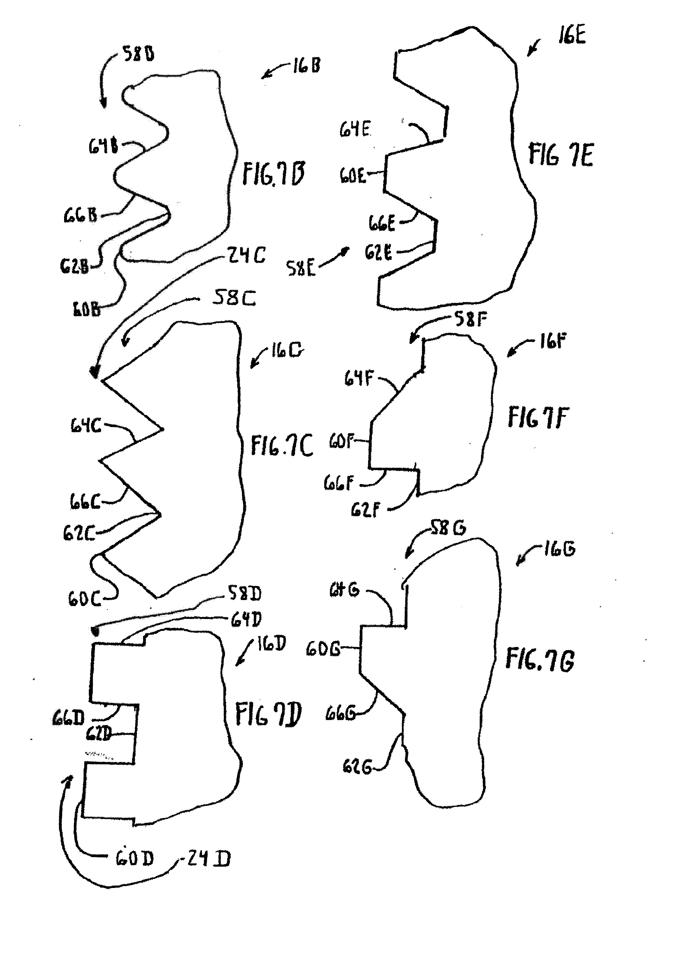

Referring now to FIG. 7, the threads 24 may have any suitable shape or thread form. For example and as shown in FIG. 7, the threads 24 may have a combination box and tapered configuration. For example and is shown in FIG. 7, the threads 50 may have any suitable shape or profile 58. For example and is shown in FIG. 7, the profile 58 may include a crest 60 and opposed root 62. A trailing surface 64 is positioned between the crest 60 and the root 62 adjacent the second end 30 of the screw 16 while the leading edge 66 is positioned between the crest 60 and root 62 adjacent the first end 20 of the screw 16.

As shown in FIG. 7, the leading edge 66 and the trailing edge 64 may be configured to provide for less force to assemble in the direction of arrow 68 than to disassemble in the direction opposed to arrow 68. Such ease of assembly and difficulty in disassembly may be accomplished as is shown in FIG. 7 by providing the trailing edge 64 with a configuration that is normal or perpendicular to the root 62 and the crest 60 while providing the leading edge 66 with chamfered or angled surface or, as is shown in FIG. 7, or with a partially angled surface between the crest 60 and the root 62.

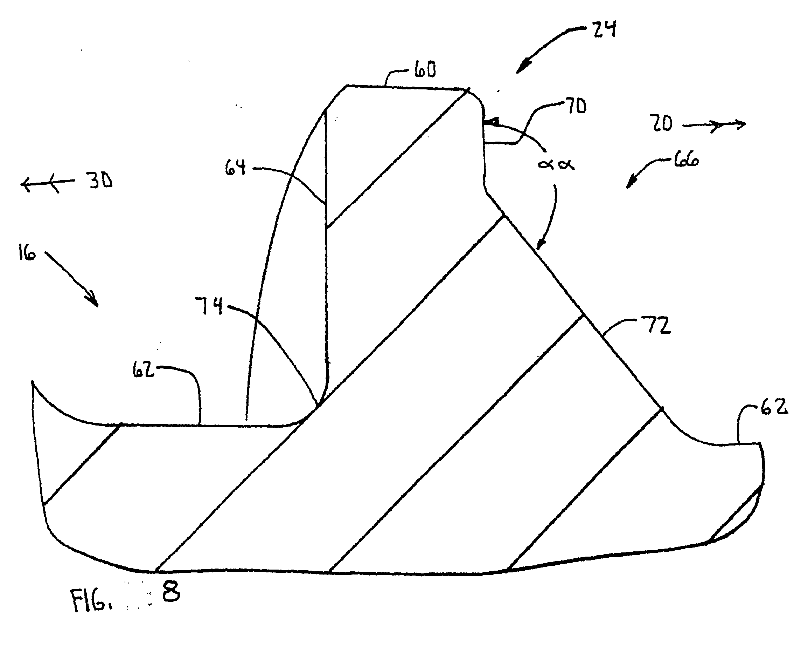

Referring now to FIG. 8, the threads 24 are shown in greater detail. The thread 24 of the screw 16 may, as is shown in FIG. 8, include the leading edge 66 such that the leading edge 66 includes normal or perpendicular portion 70 as well as an angled portion 72. The angled portion 72 provides for reduced force to assemble the screw 16 into the long bone or femur 4. The normal portion 70 and the angled portion 72 may define an angle αα between them. To minimize stress, the crest 60, the root 62, trailing edge 64, and leading edge 66 may include arcuate portions between them to minimize the stress.

Referring now to FIG. 7B-7G, alternative profile configuration for threads of the screw of the nail of the present invention as shown. Referring now to FIG. 7B, profile 58B is shown which includes arcuate roots and crest. For example and is shown in FIG. 7B, the profile 58B of the screw 16B includes an arcuate crest 60B to which the trailing angled surface 64B extends. The leading edge 66B extends likewise from the arcuate crest 60B. The profile 58B further includes an arcuate root 62B which connects with trailing surface 64B and leading surface 66B.

Referring now to FIG. 7C, yet another profile for threads for screw of the present invention is shown as screw 16C includes threads 24C having a profile 58C which include generally v-shaped threads 24C. The profile 58C includes trailing surface 64C and leading surface 66C. The root 62C and the crest 60C as shown in FIG. 7C are minimal.

Referring now to FIG. 7D, yet another profile of threads for a screw according to the present invention is shown. For example and is shown in FIG. 7D, the screw 16D includes threads 24D having a profile 58D that is blocked or rectangular. The profile 58D includes parallel and spaced apart root 62D and crest 60D. The profile 58D includes a trailing surface 64D, a spaced apart and parallel leading surface 66D. The trailing surface 64D, the leading surface 66D and are normal or perpendicular to the root 62D and the crest 60D.

Referring now to FIG. 7E, yet another embodiment of a profile of threads for a screw according to the present invention is shown. The profile 58E of threads 24E of the screw 16E has a generally truncated v-shape of that of a standard screw thread. The profile 58E includes a flat crest 60E and opposed angled trailing surface 64E and leading surface 66E. A root 62E extends from the trailing surface 64E and the leading surface 66E.

Yet another profile of threads of a screw of the present invention is shown as profile 58F. The screw 16F includes threads 24F having the profile 58F. The profile 58F includes a leading surface 66F that is normal to a crest 60F and a spaced apart parallel root 62F. The profile 58F further includes a trailing surface 64F that is positioned at an angle between the roots 62F and the crest 60F.

According to the present invention and referring now to FIG. 7G, yet another form of profile of the screw of the present invention. The screw 16G of FIG. 7G includes threads 24G defining profile 58G. The profile 58G includes a spaced apart parallel crest 60G and root 62G. The profile 58G includes a trailing surface 64G which is normal to the root 62G and the crest 60G. The profile 58G further includes a leading surface 66G which is positioned at an angle between root 62G and crest 60G.

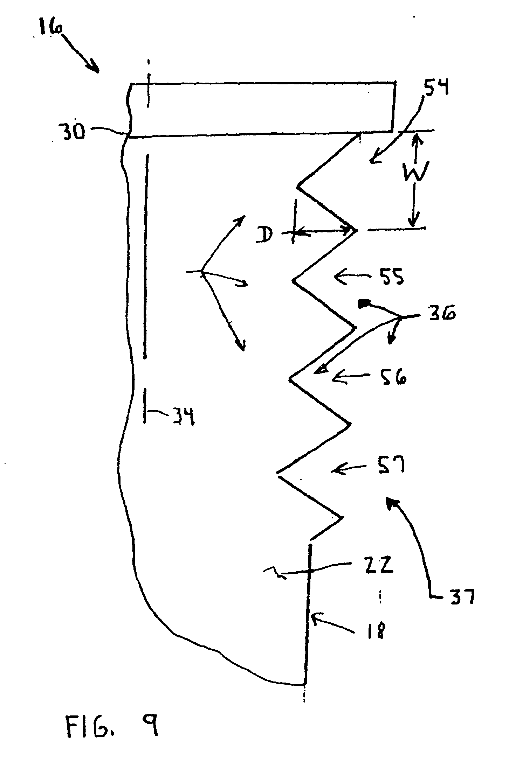

Referring now to FIG. 9, the locking feature 36 of the screw 16 is shown in greater detail. As shown in FIG. 9, the locking feature 36, may be in the form of, for example, teeth or grooves 37, including a first transverse groove or tooth 54 extending in a direction normal to a longitudinal axis 34 of a screw 16. The groove 54 may be defined for example, by a width W and a depth D. The locking feature 36 may further include a second groove 55 formed on the periphery 22 of the shank 18 of the screw 16 and spaced from the first transverse groove 54. The locking feature 36 may further include additional grooves, for example, a third groove 56 and a fourth groove 57 formed on periphery 22 of the shank 18.

In fact the groove 54, 55, 56, and 57 may form, for example, a locking feature 36 in the form of, spaced apart teeth.

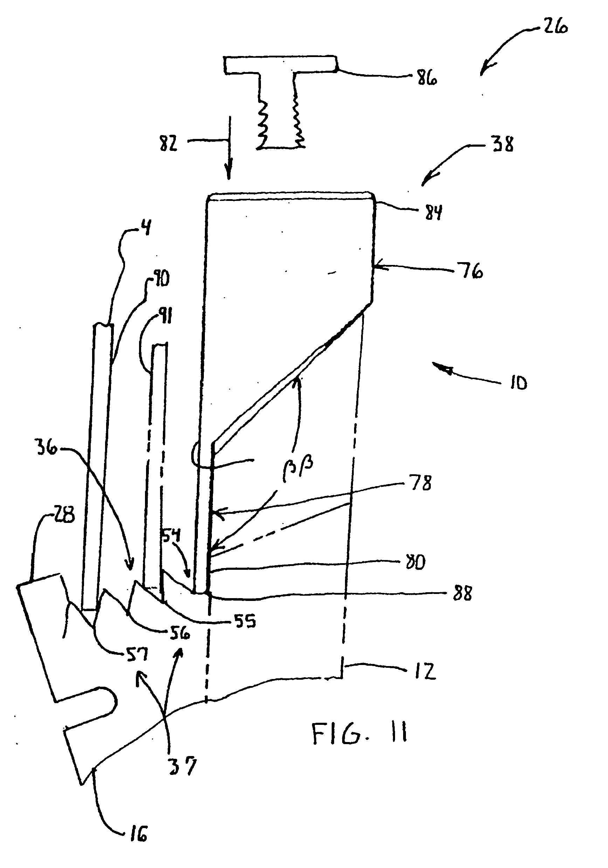

According to the present invention and referring now to FIG. 11, a locking component for use in securing the screw 16 to the intramedullary nail 12 to form the intramedullary nail assembly 10 is shown as locking component or sleeve 38. The sleeve 38 includes a first portion or sleeve portion 76 for cooperation with the nail 12. The sleeve 38 further includes a second portion 78 for securing the sleeve 38 to the screw 16.

The first portion 76 of the sleeve 38 may be adapted to slidably fit over the portion of the nail 12. The second portion 78 of the sleeve 38 may include a tab 80 extending from end 84 of the sleeve portion 76. The tab 80 may be adapted to cooperate with the screw 16.

As shown in FIG. 11, the sleeve portion 76 defines a passageway 82 in the end 84 of the sleeve portion 76. The passageway 82 is utilized for attaching a fastener, for example, a fastener in the form of screw 86 (see FIG. 2). The fastener 86 is utilized to secure locking component or sleeve 38 to the nail 12.

According to the present invention and as is shown in FIG. 11, the nail assembly 10 includes securing means 26 in which the screw 16 may be positioned in a locked position relative to the nail 12. The tab 80 of the second portion 76 of the sleeve 38 mates with one of the teeth 37 of the screw 16. For example and as shown in FIG. 11, one of the teeth 37, for example, tooth or groove 54 cooperates with the tab 80 locking the screw 16 with respect, to the nail 12. The screw 16 fixably secures the sleeve 38 to the nail 12 and to the screw 16 thereby providing a rigid construction.

Shank 18 of the screw 16 may be positioned along the transverse opening 14 of the nail 12 in any suitable position. The screw 16 may, as is shown in FIG. 11, be positioned such that lip 28 of screw 16 is positioned against cortical bone 90, of long bone 4. It should be appreciated that if the bone 4 is smaller (as shown in phantom as 91) a different tooth 37, for example third groove or tooth 56, may be engaged with tab 80 to position the cortical bone 90 in alignment with the lip 28.

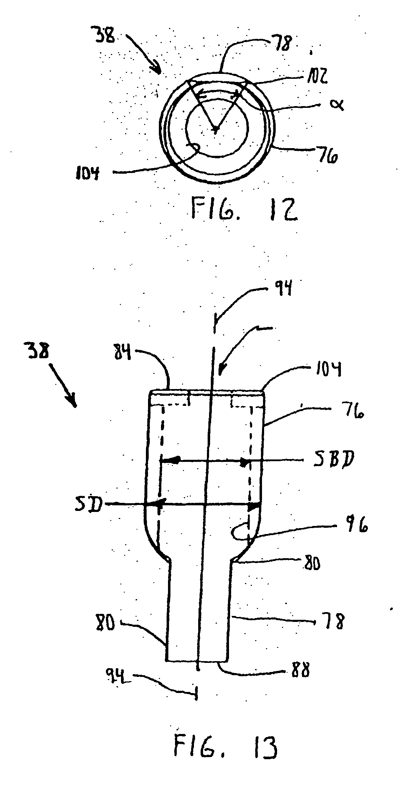

Referring now to FIGS. 12 and 13, the sleeve 38 is shown in greater detail. The sleeve 38 includes sleeve portion 76 as well as tab portion 78 extending from end 80 of the sleeve portion 76. The sleeve portion, as is shown in FIG. 12 and 13, includes an outer periphery 92 which may have any shape and for simplicity is generally cylindrical and defined by a sleeve diameter SD concentric with sleeve centerline 94. The sleeve 38 further includes a sleeve inner surface 96. The sleeve inner surface 96 may have any suitable shape and may, as is shown in FIGS. 12 and 13, be defined by diameter SBD and be slidably fitted to outer surface 98 of the nail 12 (see FIG. 2).

Referring again to FIG. 13, end 88 of the tab portion 78 of the sleeve portion 38 may have any suitable shape capable matting with teeth 37 of the screw 16. For example and as is shown in FIGS. 12 and 13, the end 88 is defined by end face 100 extending from end 88 as well as inner wall 102 of the tab portion 78. The tab portion 78 extends around the periphery of the sleeve 38 at angle α extending from sleeve centerline 94.

To fixably secure the sleeve 38 to the nail 12, the sleeve 38 may include a lip 104 positioned from end 84 of the sleeve portion 76 of the sleeve 38.

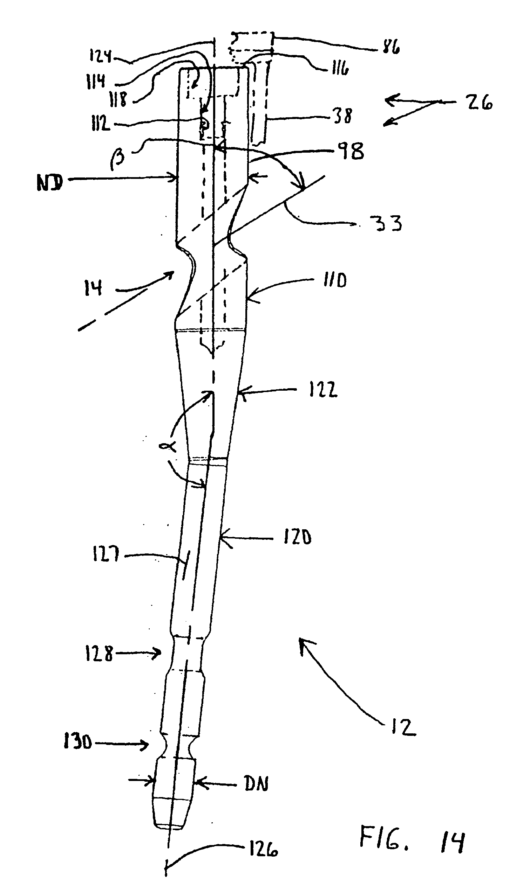

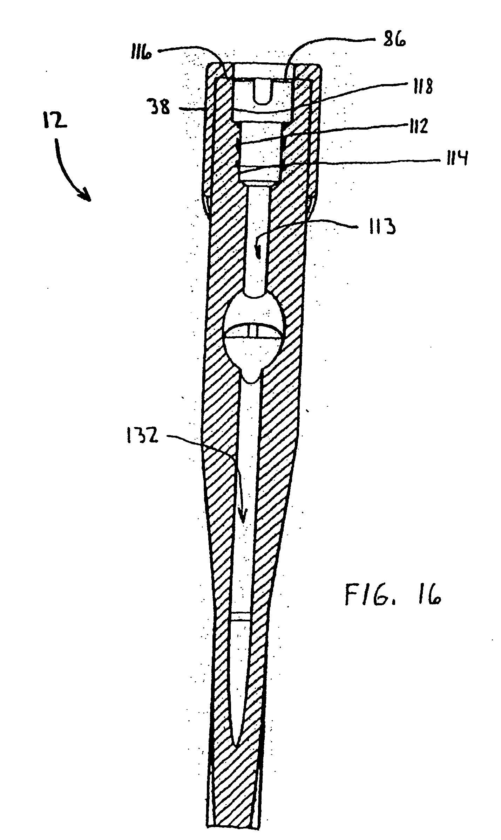

Referring now to FIG. 14-16, the intramedullary nail 12 of the nail assembly 10 is shown in greater detail. The intramedullary nail 12 has any suitable shape capable of fitting into the medullary canal of a long bone. For example and as is shown in FIG. 14, the intramedullary nail 12 may include a proximal portion 110. The proximal portion 110 includes an outer portion 98 for cooperation with sleeve 38.

The outer surface 98 of the proximal portion 110 may have any suitable shape and may for example, as is shown in FIG. 14, have a general cylindrical shape defining nail diameter ND. The proximal portion 110 of the nail 12 may include the oblique aperture 14. The oblique aperture 14 may for angle β between oblique opening centerline 33 and longitudinal centerline 124 of nail 12. The angle β is shown for screw 16 to fit into head of femur 4. The intramedullary nail 12 may include any means for securing the sleeve 38 to the nail 12. The sleeve 38 may be secured to the nail 12 by means of, for example, screw 86.

Screw 86 is secured to the nail 12 in any suitable fashion. As is shown in FIG. 14, the screw 86 may be threadably secured to the nail 12 by means of internal threads 112 formed on, small counterbore 114 formed in the proximal end 116 of the proximal portion 110 of the nail 12. Nail 12 as shown in FIG. 14, includes a large counterbore 118 positioned between the small counterbore 114 and the end 116 of the proximal portion 110 of the intramedullary nail 12.

The intramedullary nail 12 may include a distal portion 120 positioned distally from the proximal portion 110 of the nail 12. The distal portion 120 of the nail 12 may extend directly from the proximal portion 110 of the nail 12 or may, as is shown in FIG. 14, be positioned adjacent to a transition portion 122 of the nail 12. Transition portion 122 may be positioned as shown in FIG. 14 between the proximal portion 110 and the distal portion 120 of the intramedullary nail 12.

The proximal portion 110 of the nail 12 defines a proximal longitudinal centerline 124. Similarly, the distal portion 120 of the nail 12 defines a distal intramedullary centerline 126. Centrelines 124 and 126 may be coincident or may, as shown in FIG. 14, be spaced apart or form an angle therebetween. As shown in FIG. 14, the distal centerline 126 formed an angle in the proximal centerline 124 of, for example an angle αα. The angle αα may be determined to cooperate with the anatomical angle, bend or arch of the intramedullary canal of a long bone, for example, that of the femur.

The distal portion 120 of the nail 12 may define an outer surface 127. The outer surface 127 of the distal portion 120 may, as is shown in FIG. 14, be smaller than that of the outer surface 98 of the proximal portion 110. The differences in the outer surfaces 98 and 127 corresponds to the shape of the medullary canal of the long bone. For example and as shown in FIG. 14, the outer surface 127 is smaller than the outer surface 98.

The outer surface 127 may be any shape comparable and compatible with that of the medullary canal. For simplicity and to conform to the shape of the medullary canal the outer surface 127 may be cylindrical or have a round cross-section defined by a diameter DN that may be concentric with the distal centerline 126.

The distal portion 120 of the intramedullary nail may include a distal cross-hole 128 for cooperation with a screw (not shown) to provide for distal support for the intramedullary nail. The distal portion 120 of the intramedullary nail 12 may further include a second distal cross-hole 130 for cooperation with an additional screw (not shown) to provide for additional support for the nail 12 to the long bone 4 (see FIG 1).

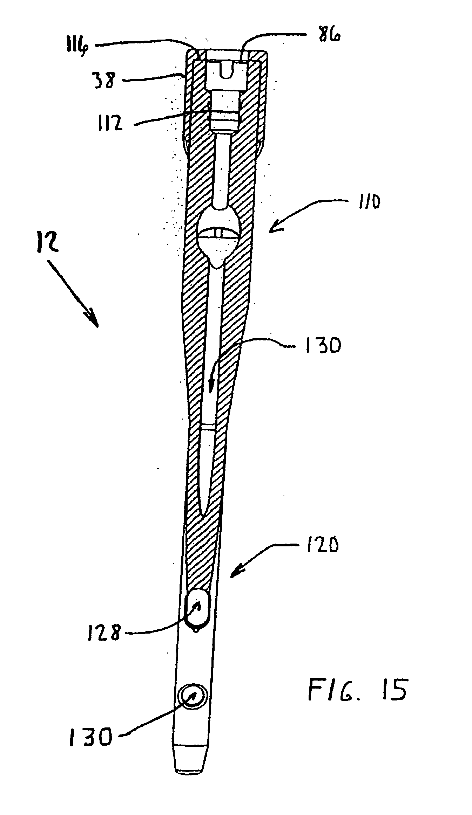

The intramedullary nail 12 may be cannulated or solid. For example and as shown in FIG. 15, the intramedullary nail 12 may include a longitudinal opening 130 extending as shown in FIG. 15 from proximal end 116 of the nail 12. The longitudinal opening 130 may extend the full length of nail 12 of the longitudinal opening or may, as is shown in FIG. 15, extend partially longitudinally and centrally in the nail 12. As shown in FIG. 15, the distal portion 120 of the nail 12 may have a generally solid cross-section.

Continuing to refer to FIGS. 14 and 15, the cross-holes 128 and 130 of the nail 12 may have, for example, a circular cross section or may have an oval cross section. For example and is shown in FIG. 15, the first distal cross-hole 128 may have an elongated or oval shape. Conversely, the second distal cross-hole 130, may have a circular cross section or may be generally cylindrical. An oval or elongated distal cross hole such as that of distal cross-hole 128 may allow for axial movement of intramedullary nail 12 in the medullary canal with the screw in position in the long bone 4.

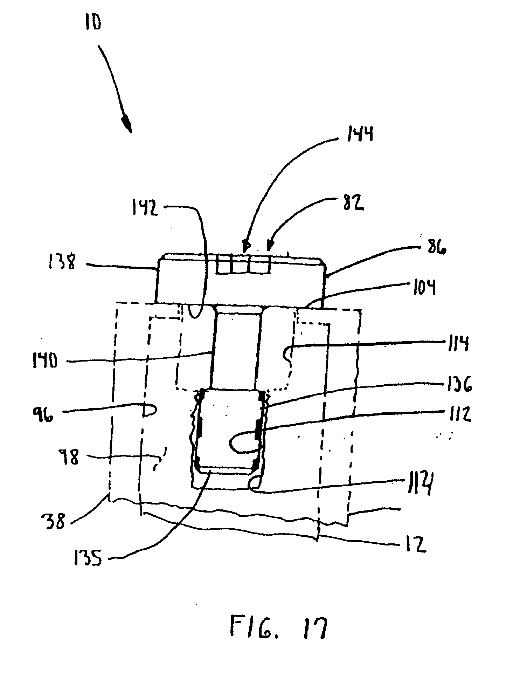

Referring now to FIG. 17, the screw 86 of the nail assembly 10 is shown in greater detail. The screw 86 is used to secure the sleeve 38 to the nail 12. The screw 86 as is shown in FIG. 17 may include a stem 135 defining external threads 136 thereon. The head 138 may extend directly from the stem 134 or may extend from a recessed shank 140 positioned between the head 136 and the stem 134. The external threads 136 on the stem 134 of the screw 86 may cooperate with internal threads 112 formed in small counterbore 112 of the nail 12.

The head 138 of the screw 86 may include a support face 142 which cooperates with lip 104 of the sleeve 38. The support face 142 of the head 138 is utilized to secure the sleeve 38 between the proximal end 116 of the nail 12 and the screw 86. The head 138 of the screw 86 may includes means in the form of, for example, a recessed socket head 144 for the tightening of the external threads 136 of the screw 86 to the internal threads 112 of the nail 12. It should be appreciated that the screw 86 may alternatively have an external socket head, a slot or other features for rotating the screw 86 with respect to the nail 12.

Referring again to FIG. 2, the nail assembly 10 may be made from components made of any suitable, durable materials that are compatible with the human anatomy. For example and is shown in FIG. 2, the nail 12, the fastener 16, the sleeve 38 and screw 86 may all be made of a suitable, durable material capable of compatibility with the human body. For example, the nail 12, the fastener 16, the sleeve 38 and screw 86 may be made of a plastic, a metal, or a composite material. Typically the nail 12, the fastener 16, the sleeve 38 and screw 86 are made of a metal. If made of a metal, the nail 12, the fastener 16, the sleeve 38 and the screw 86 may be made of, for example, a titanium alloy, a cobalt chromium alloy, or a stainless alloy. Titanium alloys are often used for the components of the nail assembly 10.

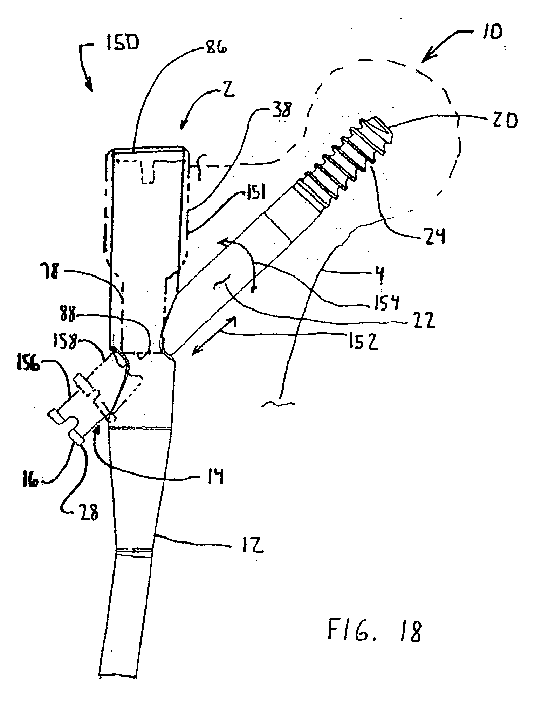

Referring now to FIG. 18-20, the nail assembly 10 may be assembled and utilized in three separate distinct modes. For example and referring now to FIG. 18, first mode 150 of the nail 10 is shown. In the first mode 150 as is shown in FIG. 18, the end 88 of the tab portion 78 of the sleeve 38 is positioned spaced from the lag screw 16.

The first mode 150 may take two separate distinct versions. For example, the nail assembly 10 in the first embodiment 150 may be absent both the sleeve 38 and the screw 86. Alternatively and is shown in FIG. 18, the first mode 150 of the nail assembly 10 may be configured such that the tab portion 78 of the sleeve 38 is rotated for example, 90° out of position from the lag screw 16 as shown in phantom as sleeve position 151. In this rotated 90° out of position location, the sleeve 38 is spaced from the lag screw 16 and may not serve to restrict the motion of the lag screw 16. The screw 86 is used to secure the sleeves 38 in the rotated position. As can be see in FIG. 18, the lag screw 16 is free to move longitudinally in the direction of longitudinal arrow 152 and rotatably in the direction of rotating arrow 154 within the transverse opening 14 of the intramedullary nail 12. As shown in FIG. 18, the lag screw 16 may freely move from first position 156 shown in solid to second position 158 as shown in phantom.

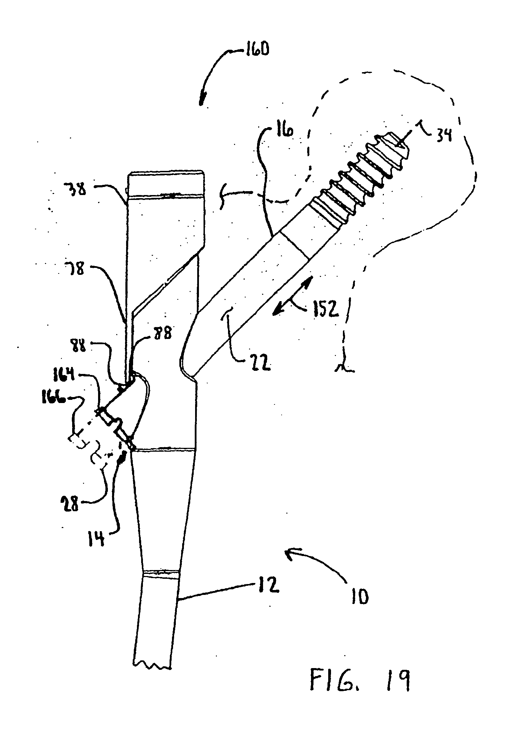

Referring now to FIG. 19, the nail assembly 10 is shown in second mode 160. When the nail assembly 10 is in second mode 160 as shown in FIG. 19, the end 88 of the tab portion 78 of the sleeve 38 is in alignment with the flat 40 formed on the periphery 22 of the lag screw 16. The lag screw 16 is thus permitted to transverse the opening 14 of the nail 12 in the direction of arrows 152. The lag screw 16 is not permitted to rotate about longitudinal centerline 34 of the lag screw 16. As shown in FIG. 19, the lag screw 16 may be translated from first position 164 shown in solid to second position 166 as shown in phantom.

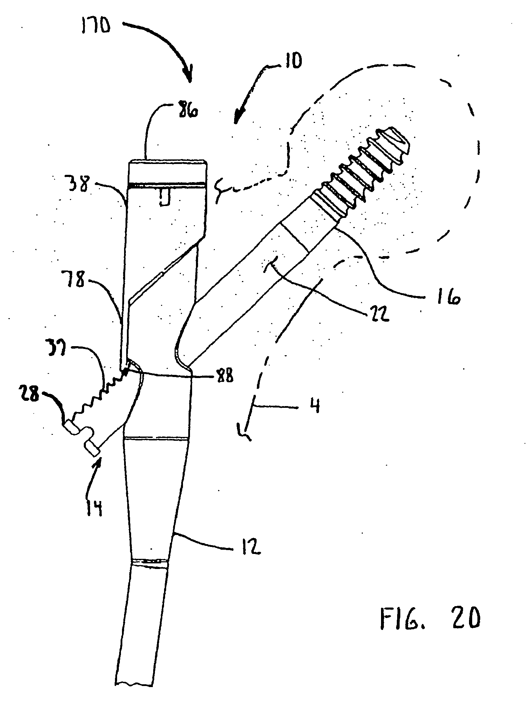

Referring now to FIG. 20, third mode 170 of the intramedullary nail assembly 10 of the present invention is shown in greater detail. The end 88 of the tab portion 78 of the sleeve 38 is engaged in teeth 37 formed on periphery 22 of the lag screw 16. The sleeve 38 prohibits the lag screw 16 from either rotation or translation in transverse opening 14 of the intramedullary nail 10. Thus the intramedullary nail 10 in the third mode 170 of FIG. 20 is a locked or constrained construction.

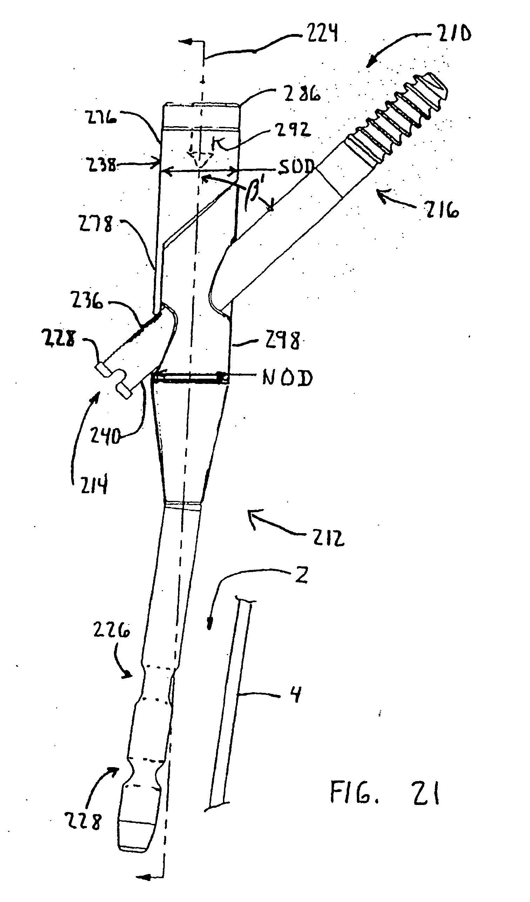

Referring now to FIGS. 21-23, yet another embodiment of the present invention is shown as intramedullary nail assembly 210. The intramedullary nail assembly 210 includes an intramedullary nail 212 that is similar to the nail 12 of FIG. 1 except that the nail 212 includes a nail outer surface 298 which is cylindrical and defined by a nail outer diameter NOD.

Referring now to FIG. 21, the nail 212 includes oblique opening 214 positioned at an angle β with respect to longitudinal axis 224 of nail 210. The opening 214 is similar to the transverse opening 14 of the nail 112 of FIG. 1. The nail 212 may as shown in FIG. 21, include a first distal cross-hole 226 and a second distal cross-hole 228.

The intramedullary nail assembly 210 further includes a screw 216 similar to the screw 16 of the nail assembly 12 of FIG. 1. The screw 216 includes a lip or head 228 and a plurality of spaced apart teeth 236. A flat 240 is positioned on the screw 216 diametrically opposed to the teeth 236.

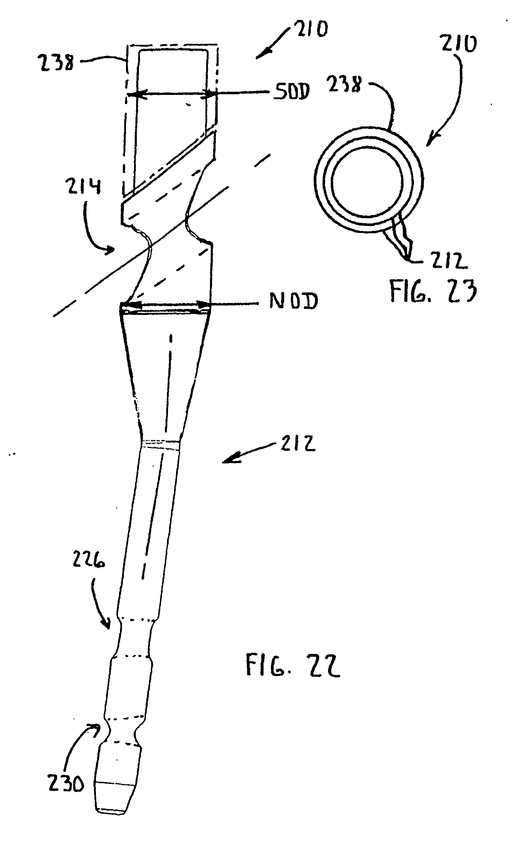

Referring now to FIG. 22, the intramedullary nail assembly 210 further includes a sleeve 238 similar to sleeve 38 of the nail assembly 10 of FIG. 1. The sleeve 238 includes a sleeve portion 276 and a tab portion 278. The tab portion 278 may cooperate with the teeth 236 and, alternatively, with flat 240. Sleeve 238 is different than the sleeve 38 of the nail 10 of FIG. 1 in that the sleeve 238 has an outer periphery 292 which is cylindrical and may be defined by diameter SOD.

The sleeve 238 is different than sleeve 38 in that the sleeve diameter SOD is designed to be similar to the diameter NOD of nail outer surface 298. The nail 210 may be more easily inserted into the intramedullary canal 2 in that the nail outer surface 298 and the sleeve outer surface 292 are in alignment to each other. The intramedullary nail assembly 210 further includes a screw 286 for securing the sleeve 238 to the nail 212 similar to the screw 16 of the nail assembly 10 in FIG. 1.

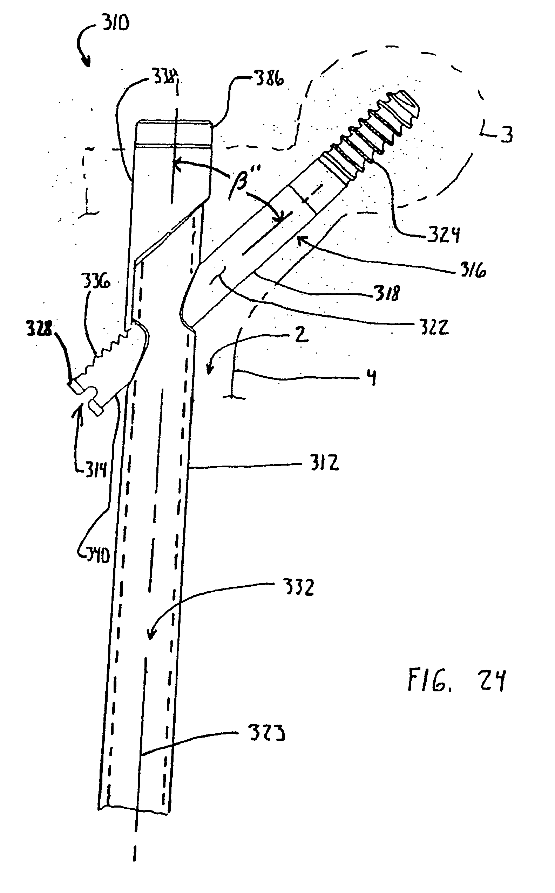

Referring now to FIG. 24, yet another embodiment of the present invention is shown as nail assembly 310. The nail assembly 310 is similar to the nail assembly 10 of FIG. 1 except that the intramedullary nail assembly 310 includes an intramedullary nail 312 that is different than the nail 12 of the nail assembly 10 of FIG. 1. The intramedullary nail 312 is hollow or cannulated.

The nail 312 as shown in FIG. 24 is straight and is uncurved or bent as is the nail 12 of the nail assembly 10 of FIG. 1. The nail 312 includes a central cannula or opening 332 which is concentric to longitudinal axis 323 of the nail 312.

The nail assembly 310 further includes a screw 316 similar to the screw 16 of nail assembly 10 of FIG. 1. The screw 316 includes a shank 318 defining a periphery 322. The screw 316 includes threads 324 as well as an opposed lip or head 328. The screw 316 includes a plurality of teeth 336 formed on the periphery 322 of the screw 316. A flat 340 is positioned opposed to the teeth 336.

The screw 316 fittably cooperates in the oblique aperture 314 formed in the nail 312. The oblique aperture 314 is shown in FIG. 24 to be angled at angle β" with respect to the longitudinal axis 323. The angle β" is selected to provide for the screw 316 to be fitted within the head 3 of the long bone or femur 4.

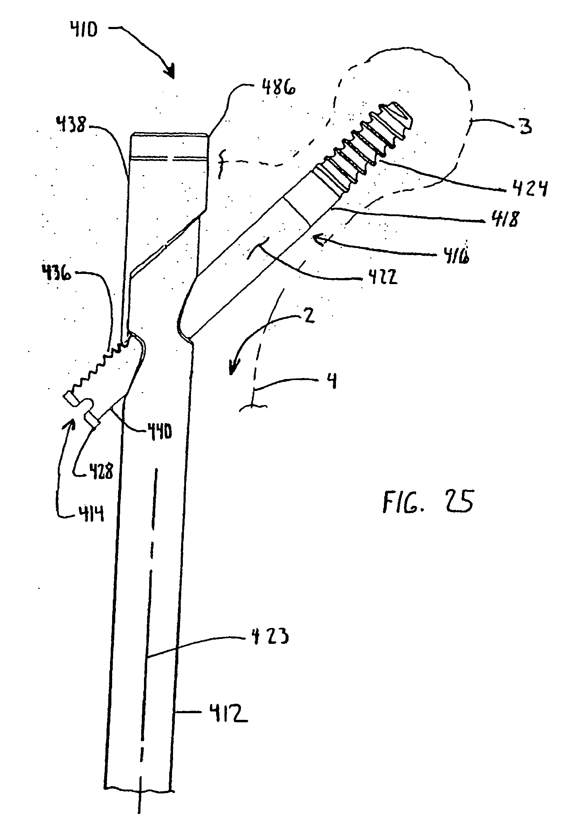

Referring now to FIG. 25, yet another embodiment of the present invention is shown as intramedullary nail assembly 410. The intramedullary nail assembly 410 is similar to the nail assembly 310 of FIG. 24 except that the intramedullary nail assembly 410 includes an intramedullary nail 412 that is solid or is not cannulated. Nail 412 is adapted to be fitted within medullary canal 2 of the femur 4.

The intramedullary nail 412 includes an oblique opening 414 positioned at an angle ββ" with respect to longitudinal axis 423 of the nail 412. The intramedullary nail assembly 410 further includes a screw 416 similar to the screw 16 of the nail assembly 10 of FIG. 1. The screw 416 includes a shank 418 defining a periphery 422 of the shank 418. Threads 424 are formed on the periphery 422 of the screw 416. The threads 424 are designed to engage head 3 of the femur 4.

The screw 416 further includes a plurality of teeth 436 formed on the periphery 422 of the screw 416. A flat 440 is formed on screw 416, opposed to the teeth 436. The screw 416 may include a lip or head 428.

The intramedullary nail assembly 410 may further include a sleeve 438 for selective engagement with the teeth 436 or the flat 440. The nail assembly 410 may include a cap 486 for securing the sleeve 438 to the nail 412.

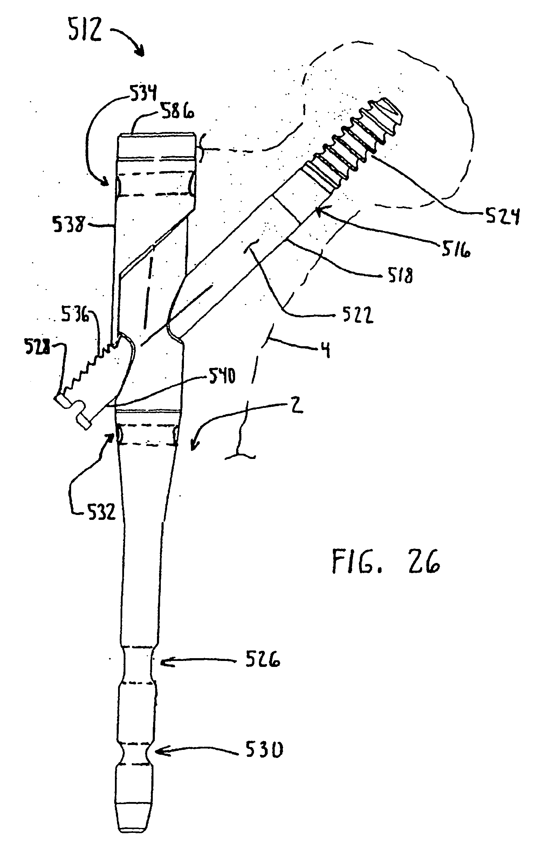

Referring now to FIG. 26, yet another embodiment of the present invention is shown as intramedullary nail assembly 510, which is similar to the nail assembly 10 of FIG. 1 except that the intramedullary nail assembly 512 of FIG. 26 includes an intramedullary nail 510 in the form of a universal nail. A universal nail is an intramedullary nail to be utilized in a number of indications. Therefore, an intramedullary nail that is a universal nail includes, as is shown in FIG. 26, additional openings for receiving additional screws.

The intramedullary nail 510 may include, for example, a first distal cross-hole 526 and a second distal cross-hole 530. Further the intramedullary nail 510 may include a first proximal cross-hole 532 and a second proximal cross-hole 534.

The intramedullary nail 510 may be hollow, solid or include a partial central opening. As shown in FIG. 26 the intramedullary nail is shown as a solid. It should be appreciated that the intramedullary nail 510 may likewise be cannulated. The intramedullary nail 510 may include an oblique opening 514 for cooperation with lag screw 516.

The intramedullary nail assembly 510 further includes the lag screw 516 similar to the lag screw 16 of the nail assembly 10 of FIG. 1. The screw 516 includes a periphery 522 defining threads 524 as well as teeth 536 and opposed flat 540. The screw 516 may further include a lip or head 528.

The nail assembly 510 may further include a sleeve 538. The nail assembly 510, screw 516 and sleeve 538 may be alternatively configured such that the screw 516 engages the teeth 536, such that the screw 516 engages the flat 540; and such that there is clearance between the screw 516 and the nail 510. The nail assembly 512 may further include a cap or screw 586 for securing the sleeve 538 to the nail 510.

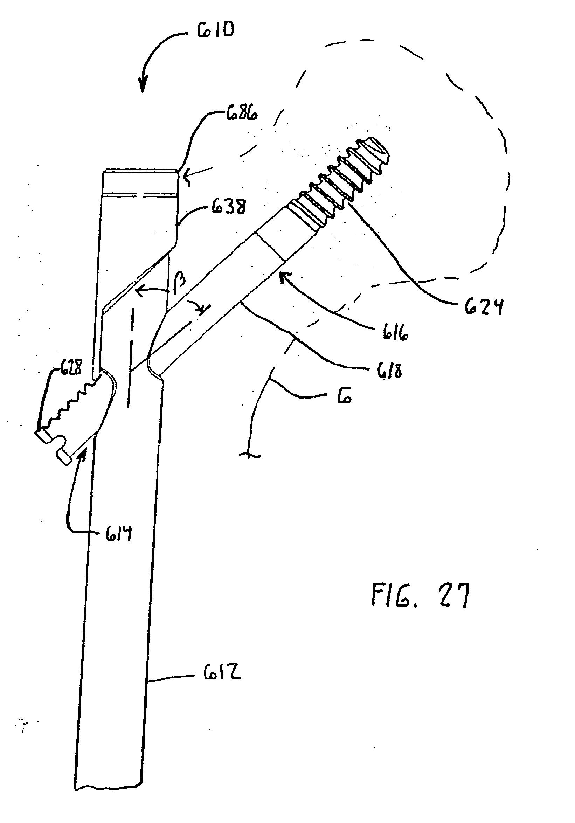

Referring now to FIG. 27, yet another embodiment of the present invention is shown as intramedullary nail assembly 612. The intramedullary nail 612 is similar to the nail assembly 12 in FIG. 1 except that the intramedullary nail 612 is adapted for use with humerus 6. The intramedullary nail assembly 612 includes an intramedullary nail 612 which defines an oblique opening 614 therein. The intramedullary nail assembly 612 further includes a screw 616 for cooperation with the transverse opening 614 of the intramedullary nail 612. The screw 616 is similar to the screw 16 of the nail assembly 10 of FIG. 1. The screw 616 includes a shank 618 which defines threads 624 as well as a head or lip 628. A sleeve 638 is used to selectively secure the screw 616 to the nail 612. A cap or screw 686 is used to secure the sleeve 638 to the intramedullary nail 612.

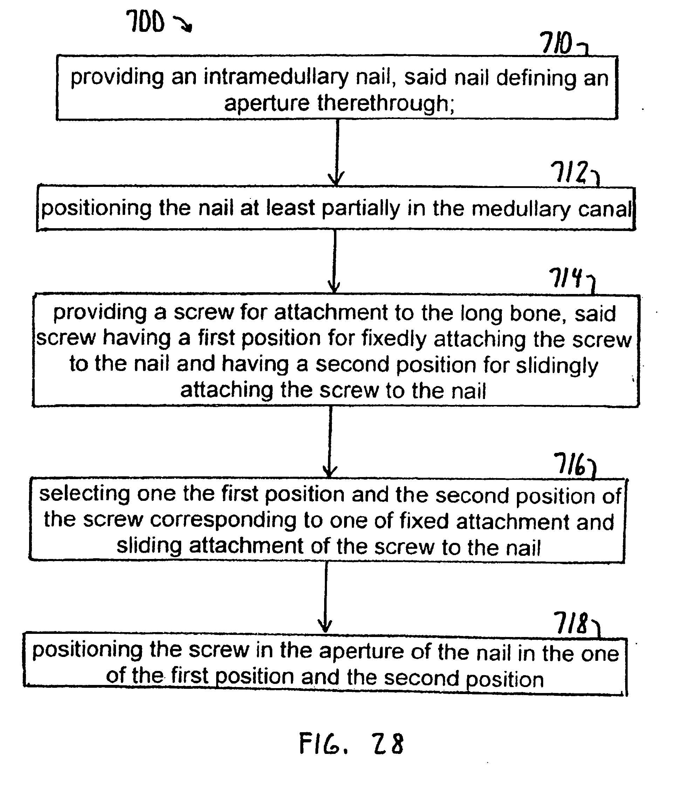

In FIG. 28 surgical method 700 is shown, which is not a part of the current invention. The surgical method 700 represents a method for performing trauma surgery on a long bone. The method 700 includes first step 710 of providing an intramedullary nail defining an aperture through the nail. The method 700 includes a second step 712 of positioning the nail partially in the medullary canal. The method 700 further includes a third step 714 of providing a screw for attachment to the long bone. The screw has a first position for fixably attaching the screw to the nail and a second position for slidably attaching the screw to the nail. The method 700 further includes a fourth step 716 of selecting one of the first positions and the second position of the screw, corresponding to one of the fixed attachments and the sliding attachment to the screw to the nail. The method 700 further includes a fifth step 718 of positioning the screw in the aperture of the nail in one of the position and the second position.

## Claims

1. An intramedullary nail assembly (10) for use in a medullary canal (2) of a long bone (4), the assembly (10) comprising:
a nail (12) for positioning at least partially in the medullary canal (2), the nail (12) defining an aperture (14) extending through it;
a screw (16) which is adapted to be positioned in the aperture (14) of the nail (12), the screw (16) having a shank (18) defining an end (20) and a periphery (22) thereof, a portion of the periphery (22) defining a thread (24); and
means (26) for securing the screw (16) to the nail (12); wherein the means (26) for securing the screw (16) to the nail (12) comprises a locking component in the form of a sleeve (38) which can be fitted over at least a portion of the nail (12);
**characterised in that** said sleeve has a tab (78) which extends from an end of the sleeve (38) to cooperate with a feature on the periphery (22) of the screw to prevent rotation of the screw (10) with respect to the nail (12).

2. The nail assembly (10) as in claim 1, wherein the feature on the periphery (22) of the screw (16) comprises at least one tooth (37) extending transverse to the longitudinal axis (34) of the screw (16) such that an end of the tab (78) can engage the tooth (37) preventing the screw (16) from rotating and translating with respect to the nail (12).

3. The nail assembly (10) as in claim 2, further comprising a series of spaced apart teeth (37) wherein said teeth (37) extend transverse to the longitudinal axis (34) of the screw (12)

4. The nail assembly (10) as in any one of claims 1 to 3, wherein the feature on the periphery (22) of the screw (16) comprises or further comprises a flat portion (40).

5. The nail assembly (10) as in claim 4 when dependent upon claim 2, wherein the flat portion (40) is diametrically opposed to the or each tooth (37).

6. The nail assembly (10) as in any one of the claims 1 to 5, which includes a fastener (86), and in which the sleeve (38) defines a passage (96) in the end thereof for receiving the fastener (86) and the nail (12) includes a fastener feature (112) formed in the nail (12) for cooperation with the fastener (86).

7. The nail assembly (10) as in claim 6, wherein the sleeve (38) further comprises a lip (104) arranged to be secured between a support surface (142) of the fastener (86) and the nail (12).

8. The nail assembly (10) as in claim 1, in which the nail (12) further defines a longitudinal opening (130) therethrough.

9. The nail assembly (10) as in claim 8, in which the nail (12) further comprises internal threads (112).

10. The nail assembly (10) as in claim 1, in which the nail (12) defines a nail centerline (94), and the aperture (14)of the nail (12) defines an aperture centerline (33), and in which the angle between nail centerline (94) and the aperture centerline (33) is an acute angle.

11. The nail assembly (10) as in claim 1, in which the nail (12) further defines a second aperture (128) through the nail (12).

12. The nail assembly (10) as in claim 1, in which the screw (16) further comprises a lip (28) extending from the shank (18) and opposed to the first mentioned end.

13. The nail assembly (10) as in claim 1, in which the screw (16) is cannulated.

## Patentansprüche

1. Im Markraum verlaufende Nadelanordnung (10) zur Verwendung in einem Markkanal (2) eines langen Knochens (4), wobei die Anordnung (10) aufweist:
einen Nagel (12) zum Positionieren wenigstens teilweise in dem Markkanal (2), wobei der Nagel (12) einen Durchlass (14) definiert, der sich durch ihn erstreckt;
eine Schraube (16), die dazu ausgelegt ist, in dem Durchlass (14) des Nagels (12) angeordnet zu werden, wobei die Schraube (16) einen Schaft (18) hat, der ein Ende (20) und ihren Umfangsbereich (22) definiert, wobei ein Teil des Umfangsbereiches (22) ein Gewinde (24) definiert; und
ein Mittel (26) zum Sichern der Schraube (16) an dem Nagel (12), wobei das Mittel (26) zum Sichern der Schraube (16) an dem Nagel (12) eine verriegelnde Komponente in der Form einer Hülse (38) aufweist, die über wenigstens einen Teil des Nagels (12) gesetzt werden kann;
**dadurch gekennzeichnet, dass** die Hülse einen Ansatz (78) hat, der sich von einem Ende der Hülse (38) erstreckt, um mit einem Merkmal auf dem Umfangsbereich (22) der Schraube zusammenzuwirken, um die Drehung der Schraube (10) in Bezug auf den Nagel (12) zu verhindern.

2. Nagelanordnung (10) nach Anspruch 1, bei der das Merkmal auf dem Umfangsbereich (22) der Schraube (16) wenigstens einen Zahn (37) aufweist, der sich quer zu der Längsachse (34) der Schraube (16) derart erstreckt, dass ein Ende Ansatzes (78) am Zahn (37) angreifen kann, so dass verhindert wird, dass sich die Schraube (16) in Bezug auf den Nagel (12) dreht und verschiebt.

3. Nagelanordnung (10) nach Anspruch 2, weiter mit einer Anzahl beabstandeter Zähne (37), wobei sich die Zähne (37) quer zu der Längsachse (34) der Schraube (12) erstrecken.

4. Nagelanordnung (10) nach einem der Ansprüche 1 bis 3, bei der das Merkmal auf dem Umfangsbereich (22) der Schraube (16) einen flachen Bereich (40) aufweist oder weiter aufweist.

5. Nagelanordnung (10) nach Anspruch 4, soweit er von Anspruch 2 abhängig ist, bei der der flache Bereich (40) dem oder jedem Zahn (37) diametral gegenüberliegt.

6. Nagelanordnung (10) nach einem der Ansprüche 1 bis 5, die ein Befestigungselement (86) umfasst, und bei der die Hülse (38) in ihrem Ende zum Aufnehmen des Befestigungselementes (86) einen Durchgang (96) definiert, , und der Nagel (12) ein Befestigungselement-Merkmal (112) umfasst, das in dem Nagel (12) zum Zusammenwirken mit dem Befestigungselement (86) gebildet ist.

7. Nagelanordnung (10) nach Anspruch 6, bei der die Hülse (38) weiter eine Lippe ( 104) aufweist, die so ausgelegt ist, dass sie zwischen einer Haltefläche (142) des Befestigungselements (86) und dem Nagel (12) zu sichern ist.

8. Nagelanordnung (10) nach Anspruch 1, bei der der Nagel (12) weiter eine durchgängige längsverlaufende Öffnung (130) definiert.

9. Nagelanordnung (10) nach Anspruch 8, bei der der Nagel (12) weiter ein Innengewinde (112) aufweist.

10. Nagelanordnung (10) nach Anspruch 1, bei der der Nagel (12) eine Nagelmittellinie (94) definiert und der Durchlass (14) des Nagels (12) eine Durchlassmittellinie (33) definiert und bei der der Winkel zwischen der Nagelmittelinie (94) und der Durchlassmittellinie (33) ein spitzer Winkel i st.

11. Nagelanordnung (10) nach Anspruch 1, bei der der Nagel (12) weiter einen zweiten Durchlass (128) durch den Nagel (12) definiert.

12. Nagelanordnung (10) nach Anspruch 1, bei der die Schraube (16) weiter eine Lippe (28) aufweist, die sich von dem Schaft (18) und gegenüber zu dem ersten genannten Ende erstreckt.

13. Nagelanordnung (10) nach Anspruch 1, bei der die Schraube (16) mit einer Kanüle versehen ist.

## Revendications

1. Ensemble de clou intramédullaire (10) destiné à être utilisé dans un canal médullaire (2) d'un os long (4), l'ensemble (10) comprenant :
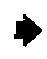 un clou (12) pour le positionnement au moins partiellement dans le canal médullaire (2), le clou (12) définissant une ouverture (14) s'étendant à travers celui-ci ;
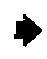 une vis (16) qui est adaptée pour être positionnée dans l'ouverture (14) du clou (12), la vis (16) ayant une tige (18) définissant une extrémité (20) et sa périphérie (22), une partie de la périphérie (22) définissant un filetage (24) ; et
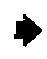 des moyens (26) pour fixer la vis (16) sur le clou (12) ; dans lequel les moyens (26) pour fixer la vis (16) sur le clou (12) comprennent un composant de blocage se présentant sous la forme d'un manchon (38) qui peut être monté sur au moins une partie du clou (12) ;
**caractérisé en ce que** ledit manchon a une languette (78) qui s'étend à partir d'une extrémité du manchon (38) pour coopérer avec une caractéristique sur la périphérie (22) de la vis pour empêcher la rotation de la vis (10) par rapport au clou (12).

2. Ensemble de clou (10) selon la revendication 1, dans lequel la caractéristique sur la périphérie (22) de la vis (16) comprend au moins une dent (37) s'étendant de manière transversale par rapport à l'axe longitudinal (34) de la vis (16) de sorte qu'une extrémité de la languette (78) peut mettre en prise la dent (37) empêchant la rotation et la translation de la vis (16) par rapport au clou (12).

3. Ensemble de clou (10) selon la revendication 2, comprenant en outre une série de dents (37) espacées, dans lequel lesdites dents (37) s'étendent de manière transversale par rapport à l'axe longitudinal (34) de la vis (12).

4. Ensemble de clou (10) selon l'une quelconque des revendications 1 à 3, dans lequel la caractéristique sur la périphérie (22) de la vis (16) comprend ou comprend en outre une partie plate (40).

5. Ensemble de clou (10) selon la revendication 4 lorsqu'elle dépend de la revendication 2, dans lequel la partie plate (40) est diamétralement opposée à la ou chaque dent (37).

6. Ensemble de clou (10) selon l'une quelconque des revendications 1 à 5, qui comprend une fixation (86), et dans lequel le manchon (38) définit un passage (96) dans son extrémité pour recevoir la fixation (86) et le clou (12) comprend une caractéristique de fixation (112) formée dans le clou (12) pour la coopération avec la fixation (86).

7. Ensemble de clou (10) selon la revendication 6, dans lequel le manchon (38) comprend en outre une lèvre (104) agencée pour être fixée entre une surface de support (142) de la fixation (86) et le clou (12).

8. Ensemble de clou (10) selon la revendication 1, dans lequel le clou (12) définit en outre une ouverture longitudinale (130) à travers celui-ci.

9. Ensemble de clou (10) selon la revendication 8, dans lequel le clou (12) comprend en outre des filetages internes (112).

10. Ensemble de clou (10) selon la revendication 1, dans lequel le clou (12) définit un axe central de clou (94) et l'ouverture (14) du clou (12) définit un axe central d'ouverture (33), et dans lequel l'angle entre l'axe central de clou (94) et l'axe central d'ouverture (33) est un angle aigu.

11. Ensemble de clou (10) selon la revendication 1, dans lequel le clou (12) définit en outre une seconde ouverture (128) à travers le clou (12).

12. Ensemble de clou (10) selon la revendication 1, dans lequel la vis (16) comprend en outre une lèvre (28) s'étendant à partir de la tige (18) et opposée à la première extrémité mentionnée.

13. Ensemble de clou (10) selon la revendication 1, dans lequel la vis (16) est tubulaire.
